(19) Europäisches Patentamt
European Patent Office
Office européen des brevets

(11) Veröffentlichungsnummer: **0 164 588**
**A2**

(12) **EUROPÄISCHE PATENTANMELDUNG**

(21) Anmeldenummer: 85105798.4

(22) Anmeldetag: 11.05.85

(51) Int. Cl.⁴: A 61 K 31/44

(30) Priorität: 23.05.84 DE 3419128

(43) Veröffentlichungstag der Anmeldung: 18.12.85 Patentblatt 85/51

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Posanski, Ulrich, Dr.
Ahornweg 65b
D-5064 Rösrath(DE)

(54) **Dihydropyridinpräparate und Verfahren zu ihrer Herstellung.**

(57) Die Erfindung betrifft neue Dihydropyridinpräparate in fester Form mit verbesserter Bioverfügbarkeit, enthaltend schwer lösliche Dihydropyridine und leicht lösliche Hilfsstoffe, Verfahren zu ihrer Herstellung und ihre Verwendung als Arzneimittel, insbesondere als Coronarund Blutdruckmittel.

EP 0 164 588 A2

Croydon Printing Company Ltd.

0164588

BAYER AKTIENGESELLSCHAFT
Konzernverwaltung RP
Patentabteilung

5090 Leverkusen, Bayerwerk
V (Pha)
KS/by/c

Dihydropyridinpräparate und Verfahren zu ihrer Herstellung

Die Erfindung betrifft neue Dihydropyridinpräparate in fester Form mit verbesserter Bioverfügbarkeit, enthaltend schwer lösliche Dihydropyridine und leicht lösliche Hilfsstoffe, Verfahren zu ihrer Herstellung und ihre Verwendung als Arzneimittel, insbesondere als Coronar- und Blutdruckmittel.

Es ist bereits bekannt geworden, daß Dihydropyridine sehr starke kreislaufbeeinflussende Wirkungen besitzen (vgl. britisches Patent 1 358 951). Aufgrund der schweren Löslichkeit vieler Dihydropyridine treten bei der galenischen Zubereitung von Arzneispezialitäten eine Reihe von Schwierigkeiten auf, wie aus zahlreichen Publikationen und Patentanmeldungen über spezielle Formulierungen dieser Wirkstoffe ersichtlich wird. In dem britischen Patent 1 456 618 werden z.B. feste Arzneizubereitungen beschrieben und beansprucht, welche ebenfalls eine gute Bioverfügbarkeit von schwer löslichen Dihydropyridinen gewährleisten sollen. Auch in der DT-OS 2 822 882

werden feste Arzneiformen beschrieben, bei denen durch den Einsatz bestimmter Lösungsvermittler und oberflächenaktiver Substanzen die Schwerlöslichkeit der Wirkstoffe kompensiert werden soll. Auch in der europäischen Offenlegungsschrift 1 247 soll die Resorbierbarkeit von Dihydropyridinen durch die Verwendung von Polyethylenglykol und bestimmter poröser Trägersubstanzen verbessert werden.

In der Literatur werden ebenfalls verschiedene Methoden zur Verbesserung der Bioverfügbarkeit, die unmittelbar mit einer verbesserten Löslichkeit verbunden ist, beschrieben. Neben der Änderung der chemischen Struktur, z.B. durch Einbau von löslichkeitsverbessernden Substituentengruppen sind zahlreiche Methoden beschrieben, die eine Änderung der physikalischen Eigenschaften der Wirkstoffe betreffen. So wird z.B. die Zerkleinerung von Wirkstoffpartikeln, Veränderung der Kristallstruktur, die Herstellung von Salzformen, Zugabe von Netzmitteln, Komplexbildung mit anderen Substanzen, Verwendung von biologischen Carriern, Erstellung fester Dispersionen (Copräzipitate) oder die Herstellung von Oberflächenadsorbaten vorgeschlagen (vgl. S.H. Yalkowskay, Drugs and the pharmaceutical science, 12, 135-180 (1981) und J. Polderman, Formulation and preparation of dosage forms, Elsevier/North-Holland Biomedical Press, 1977, 215-219).

Alle bisherigen Versuche, die schlechte Löslichkeit von Dihydropyridinen durch bestimmte Maßnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, z.B. porös sind, führt häufig zu Verabreichungsformen, bei denen die Präparate unerwünscht groß sind. Zur Erleichterung des Schluckens werden solche Tabletten oder Kapseln häufig in spezifische Formen wie z.B. Ellipsoide oder Längsformen überführt, was jedoch bei Präparaten mit einem Gewicht über 400 mg auch nicht immer zu befriedigenden Ergebnissen führt. Auch das häufigere Einnehmen von kleineren Präparaten stellt keine befriedigende Lösung dar.

Die vorliegende Erfindung betrifft neue feste Dihydropyridinpräparate mit verbesserter Bioverfügbarkeit, enthaltend 1 Gewichtsteil eines schwer löslichen Dihydropyridins der allgemeinen Formel (I)

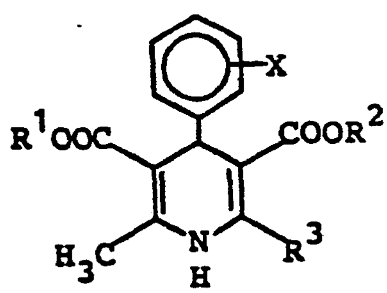

(I)

in der

$R^1$ $C_1$-$C_4$-Alkyl, gegebenenfalls substituiert durch $C_2$-$C_3$-Alkoxy,

$R^2$ $C_1$-$C_{10}$-Alkyl, gegebenenfalls substituiert durch $C_1$-$C_3$-Alkoxy, Trifluormethyl, N-Methyl-N-benzylamino,

$R^3$ $C_1$-$C_4$-Alkyl, Cyano, Hydroxymethyl und

X 2- bzw. 3-Nitro, 2,3-Dichlor, 2,3 Ringglied bestehend aus =N-O-N=,

bedeuten,

und 1,0 bis 20, vorzugsweise 2,0 bis 15 Gewichtsteile eines gut wasserlöslichen Füllstoffes und gegebenenfalls weitere übliche Hilfs- und Trägerstoffe.

Von besonderem Interesse sind erfindungsgemäße Dihydropyridinpräparate, die als gut wasserlösliche Füllstoffe Zucker und/oder Zuckeralkohole, wie z. B. Lactose, Saccharose, Fructose, Glucose, Mannit, Sorbit und/oder gut wasserlösliche Salze wie Magnesium-, Calcium-, Kalium-, Natrium-, Ammonium-Salzen, wobei das Anion z. B. Chlorid, Carbonat, Citrat, Tartrat, Lactat, Acetat, Hydrogencarbonat sein kann,enthalten.

Vorzugsweise genannt seien erfindungsgemäße Dihydropyridinpräparate enthaltend 1 Gewichtsteil eines Dihydropyridins der allgemeinen Formel I, in welcher $R^1$ und $R^2$ immer voneinander verschieden sind, insbesondere Nisoldipin, Nimodipin oder Nitrendipin und 2 bis 15 Gewichtsteile eines gut löslichen Füllstoffes, insbesondere Lactose und/oder Mannit.

Als gut wasserlösliche Füllstoffe gelten alle physiologisch unbedenklichen Stoffe deren Löslichkeit mindestens 15 g insbesondere mindestens 20 g pro 100 ml Wasser bei 25°C beträgt.

Die Herstellung der erfindungsgemäßen Kombinationspräparate erfolgt, indem man den Dihydropyridinwirkstoff und

den gut wasserlöslichen Füllstoff gemeinsam, gegebenenfalls mit bekannten Hilfsstoffen granuliert mittels eines Feuchtgranulationsverfahrens oder eines Trockenkompaktierverfahrens und das erhaltene Granulat gegebenenfalls mit mindestens einem Tablettenhilfsstoff zu Tabletten verpreßt.

Die Herstellung der erfindungsgemäß verwendbaren Dihydropyridine der allgemeinen Formel I erfolgt vorzugsweise durch Umsetzung von Ylidenverbindungen der allgemeinen Formel II

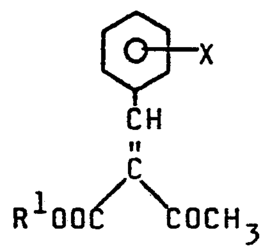

(II)

mit Enaminverbindungen der allgemeinen Formel III

$$R^3\text{-}\underset{\displaystyle NH_2}{C}\text{=CH-COOR}^2 \qquad \text{(III)}$$

wobei $R^1$, $R^2$, $R^3$ und X die für Formel I angegebene Bedeutung haben,

in Gegenwart von niederen aliphatischen Alkoholen als Lösungsmittel bei Temperaturen von 40 - 100°C.

Eine vorteilhafte Variante dieser Umsetzung liegt in der Verwendung eines geringen molaren Überschusses der Enaminverbindung der allgemeinen Formel III und einer Entfernung des sich bildenden Reaktionswassers, gegebenenfalls durch azeotrope Destillation, während der Umsetzung.

Die Ylidenverbindung der Formel II wird vorzugsweise hergestellt durch Reaktion molarer Mengen eines Keto-

carbonsäureesters mit einem aromatischen Aldehyd in niederen Alkoholen als Lösungsmittel, insbesondere Alkohol mit 1 bis 4 Kohlenstoffen, in Gegenwart von katalytischen Mengen Piperidinacetat bei Temperaturen zwischen 20 und 70°C.

Die Herstellung der Enaminverbindung der allgemeinen Formel III erfolgt vorzugsweise durch Umsetzung von Ketocarbonsäureestern und Ammoniak in niederen Alkoholen, vorzugsweise Isopropanol, bei Temperaturen zwischen 0 und 50°C.

Als Hilfsstoffe und Trägerstoffe seien beispielhaft genannt:
Zerfallsförderer wie Stärke, modifizierte Stärke, Zellulose, Zellulosederivate, quervernetztes Polyvinylpyrrolidon (PVPP), Natriumalginat und kolloidales Siliciumdioxid; Bindemittel wie Gelatine, Tragant, Glukosesirup, Stärkekleister, Polyvinylpyrrolidone (PVP), Zellulosederivate, Polyethylenglykol MG 1000-5000 (PEG), und Alginate; Schmiermittel wie Magnesiumstearat, Calciumstearat, Stearinsäure, Paraffin, Talkum, pflanzliche oder tierische Fette, Öle und Wachse, Polyethylenglykol MG 1000-5000 und Silikone.
Calciumsulfat, Calciumcarbonat, di- und tribasische Calciumphosphate, Magnesiumcarbonat, Magnesiumhydroxycarbonat, Natriumchlorid, Natrium- und Kalium-zitrate, Tartrate und Succinate, Stärke, modifizierte Stärke, Zellulose, Zellulosepulver, Zucker wie Lactose, Saccharose oder Dextrose und Zuckeralkohole wie Mannit oder Sorbit.

Von besonderem Interesse sind Hilfs-, Träger- und Füllstoffe aus der Gruppe Stärke, modifizierte Stärke wie Stärkekleister, Zellulose, PVP, PVPP, kolloidales Siliciumdioxid, Lactose, Magnesiumcarbonat, Magnesiumstearat und Calciumstearat.

Bei Kenntnis des Standes der Technik ist es ausgesprochen überraschend, daß durch die gemeinsame Granulation eines schwer löslichen Dihydropyridins mit einem der genannten Füllstoffe eine signifikante Erhöhung der Freisetzung und damit eine signifikante Verbesserung der Bioverfügbarkeit für das Dihydropyridin erreicht werden kann. Dieser erfindungsgemäße Effekt ist nicht abhängig von der Anwesenheit zusätzlicher Hilfsstoffe, die nach Kenntnis nach Standes der Technik die Freisetzungsrate erhöhen wie z.B. Komplexbildnern, oberflächenaktiven Stoffen oder wasserlöslichen Polymeren.

Als schwerlösliche Dihydropyridine seien Dihydropyridine genannt, deren Löslichkeit maximal 20 mg pro Liter Wasser bei 25°C beträgt.

Im Freisetzungstest nach der USP-Paddle-Methode unter den folgenden Bedingungen

4000 ml 0,1 N-Salzsäure

Paddle-Drehzahl 100 U/Min.

wurden nach 2 Stunden Freisetzungsraten für Nitrendipin von ca. 80 % erreicht für erfindungsgemäße Präparate (Produkt gemäß Beispiel 1). Eine entsprechende Zubereitung einer herkömmlichen Nitrendipintablette, in welcher das Nitrendipin in gleicher kristalliner Zusammensetzung eingesetzt wurde und die die gleichen Hilfsstoffe enthält, zeigt nach 2 Stunden nur eine Freisetzungsrate von ca. 40 %. Der Verlauf der Freisetzung ist aus der Tabelle 1 ersichtlich.

Tabelle 1

In vitro - Freisetzung von nitrendipinhaltigen Tabletten (USP-Paddle-Methode)

| Freisetzungszeit /h/ | kumulative Freisetzung von Nitrendipin in Prozent der eingesetzten Dosis<br>Tablette Beispiel 1 | Tablette Vergleichsbeispiel |
|---|---|---|
| 0,25 | 29 | 12 |
| 0,5 | 56 | 21 |
| 1 | 69 | 34 |
| 2 | 84 | 41 |

Vergleichsbeispiel

100 g Nitrendipin, 910 g Maisstärke, 680 g Cellulosepulver und 550 g Calciumcarbonat und 10 g Natriumlaurylsulfat werden in einem Planetenmischer 5 Minuten gemischt und mit einer wäßrigen Polyvinylpyrrolidon-Lösung (Feststoffgehalt 50 g) 10 Minuten granuliert. Die feuchte Masse wird durch ein Sieb gegeben und in einem Wirbelschichttrockner auf einen Endwassergehalt von ca. 4,5 % getrocknet. Das getrocknete Granulat wird durch Siebung (0,8 mm-Siebmaschenweite) egalisiert und intensiv mit 50 g Magnesiumstearat vermischt. Anschließend wird das Granulat zu 235 mg schweren Tabletten verpreßt.

0164588

## Ausführungsbeispiele:

### Beispiel 1

100 g Nitrendipin, 1200 g Lactose, 1280 g Maisstärke, 430 g Cellulosepulver, 350 g Calciumcarbonat und 10 g Natriumlaurylsulfate werden in einem Planetenmischer 5 Minuten gemischt und mit einer wäßrigen Polvinylpyrrolidon-Lösung (Feststoffgehalt 50 g) 10 Minuten granuliert. Die feuchte Masse wird durch ein Sieb gegeben und in einem Wirbelschichttrockner auf einen Endwassergehalt von ca. 4,5 % getrocknet. Das getrocknete Granulat wird durch Siebung (0,8 mm-Siebmaschenweite) egalisiert und intensiv mit 50 g Magnesiumstearat vermischt. Anschließend wird das Granulat zu 235 mg schweren Tabletten verpreßt. Die Tabletten können mit einem Schutzlack überzogen werden.

### Beispiel 2

50 g Nisoldipin, 2216 g Mannit, 2244 g Maisstärke, 250 g vorverkleisterte Maisstärke und 750 g Cellulosepulver werden in einem Mischgranulator mit einer wäßrigen Tween 80-Lösung (Gehalt 15 g) granuliert. Danach wird die feuchte Masse durch eine Raspel (Lochweite 4,0 mm) gegeben und in einem Umlufttrockenschrank auf ca. 3,8 % Endfeuchte getrocknet. Das getrocknete Granulat wird durch Siebung (1,25 mm-Siebmaschweite) egalisiert und intensiv mit 15 g Magnesiumstearat vermischt. Anschließend wird das Granulat zu 277 mg schweren Tabletten verpreßt.

Die Tabletten können mit einer Lichtschutzumhüllung überzogen werden. Alternativ kann das Granulat in Kapseln abgefüllt werden.

Beispiel 3

1200 g Mannit, 400 g Nitrendipin, 100 g Cellulosepulver, 1800 g Maisstärke und 40 g Natriumlaurylsulfat werden in einem Mischgranulator (z.B. Lödige MGT 30) 1 Minute gemischt und 5 Minuten lang mit Stärkekleister (Stärkegehalt 200 g) granuliert bei einer Rotordrehzahl des Granulators von 200 U/Min. Durch Zerhacker-Stufe II wird eine mittlere Teilchengröße von ca. 200 bis 300 µ erhalten. Das Granulat wird über eine Raspel (Siebeinsatz 2,0 mm Ø) gegeben und in einem Fließbetttrockner (z.B. Glatt WST 5) bis auf eine Endfeuchte von ca. 4,0 % Wasser getrocknet. Das getrocknete Granulat wird mit 100 g Magnesiumstearat in einem Mischer 10 Minuten gemischt und anschließend zu Tabletten mit einem mittleren Durchmesser von 9 mm und einem Gewicht von ca. 237 mg verpeßt.

Die erhaltenen Tabletten können zusätzlich mit einer lichtundurchlässigen Lackhülle überzogen werden, die z.B. rotes Eisenoxid als Lichtschutz enthält.

Beispiel 4

600 g Lactose, 200 g Nimodipin, 800 g Cellulosepulver, 600 g Maisstärke, 25 g Tween 80 und 50 g Magnesiumstearat werden in einem Kubusmischer gemischt und anschließend trocken kompaktiert (Alexanderwalze WP50; Kompaktierungsdruck 35 bar). Die Schülpen werden vorgebrochen, mit einem 1,25 mm-Sieb egalisiert und mit einer Mischung aus 25 g Magnesiumstearat und 200 g Maisstärke homogen vermischt. Das fertige Granulat wird zu 250 mg schweren Tabletten mit 9 mm Ø verpreßt.

Die erhaltenen Tabletten können direkt verwendet werden oder alternativ noch einen Schutzlack, bestehend aus Hydroxipropylmethylcellulose, Polyethylenglykol und Eisenoxiden oder anderen physiologisch verträglichen Farbpigmenten, erhalten.

Beispiel 5

950 g Mannit, 300 g Nisoldipin, 1255 g Maisstärke, 150 g vorverkleisterte Maisstärke und 900 g Cellulosepulver werden 8 Minuten in einem Planetenmischer (Collette MP 20) gemischt und 15 Minuten lang, nach kontinuierlicher Zugabe von 250 ml einer 2,0 %igen, wäßrigen Natriumlaurylsulfatlösung, bei erhöhter Drehzahl des Mischers granuliert. Die feuchte Masse wird geraspelt, in einem Wirbelschichttrockner auf ca. 3,8 % Endfeuchte getrocknet und durch Siebung (1,0 mm Maschenweite) egalisiert. Die Endmischung wird nach Untermischung von 75 g Magnesiumstearat erhalten und zu 247 mg schweren Tabletten verpreßt.

Die erhaltenen Tabletten können zusätzlich mit einer tageslichtundurchlässigen Hülle überzogen werden. Das Granulat kann alternativ in Hartgelatinekapseln abgefüllt werden.

Patentansprüche

1. Festes Dihydropyridinpräparat mit verbesserter Bioverfügbarkeit, enthaltend 1 Gewichtsteil eines schwerlöslichen Dihydropyridins der allgemeinen Formel (I)

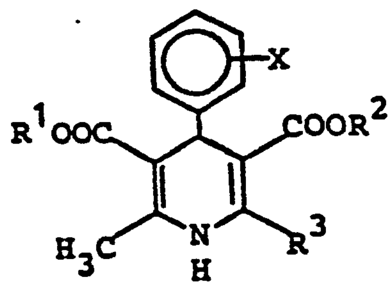

(I)

in der

$R^1$ $C_1$-$C_4$-Alkyl, gegebenenfalls substituiert durch $C_2$-$C_3$-Alkoxy,

$R^2$ $C_1$-$C_{10}$-Alkyl, gegebenenfalls substituiert durch $C_1$-$C_3$-Alkoxy, Trifluormethyl, N-Methyl-N-benzylamino,

$R^3$ $C_1$-$C_4$-Alkyl, Cyano, Hydroxymethyl und

X 2- bzw. 3-Nitro, 2,3-Dichlor, 2,3 Ringglied bestehend aus =N-O-N=,

bedeuten,

und 1 bis 20 Gewichtsteile eines gut wasserlöslichen Füllstoffes und gegebenenfalls übliche Hilfs- und Trägerstoffe.

2. Dihydropyridinpräparat gemäß Anspruch 1, enthaltend einen gut wasserlöslichen Füllstoff aus der Gruppe Lactose und Mannit.

3. Dihydropyridinpräparat gemäß Anspruch 1, enthaltend ein Dihydropyridin der allgemeinen Formel I, in welcher $R^1$ und $R^2$ immer voneinander verschieden sind.

4. Dihydropyridinpräparat gemäß Ansprüchen 1 bis 3, enthaltend 1 Gewichtsteil des schwerlöslichen Dihydropyridins und 2 bis 15 Gewichtsteile eines gut wasserlöslichen Füllstoffes.

5. Dihydropyridinpräparat gemäß den Ansprüchen 1 bis 4, enthaltend Nitrendipin, Nisoldipin oder Nimodipin.

6. Präparat gemäß den Ansprüchen 1 bis 5 in Form von Granulat.

7. Verfahren zur Herstellung von Präparaten gemäß Anspruch 1, dadurch gekennzeichnet, daß man 1 Gewichtsteil des schwerlöslichen Dihydropyridins der allgemeinen Formel (I) und 1,0 bis 20 Gewichsteile des gut wasserlöslichen Füllstoffes gegebenenfalls gemeinsam mit Hilfs- und Trägerstoffen granuliert und gegebenenfalls dieses Granulat zu üblichen Applikationsformen weiterverarbeitet.

8. Verfahren gemäß Anspruch 7, dadurch gekennzeichnet, daß man 1 Gewichtsteil Dihydropyridin und 2 bis 15

Gewichtsteile des gut wasserlöslichen Füllstoffes mit Stärke, Cellulose, PVP und Siliciumdioxid in einem Granulator wäßrig granuliert und das erhaltene Granulat gegebenenfalls in eine übliche Applikationsform überführt.

9. Verwendung von Dihydropyridinpräparaten gemäß Anspruch 1 bei der Bekämpfung von Kreislauferkrankungen.

10. Verfahren zur Herstellung von Präparaten gemäß Ansprüchen 1 bis 6, dadurch gekennzeichnet, daß man Ylidenverbindungen der allgemeinen Formel II, in welcher $R^1$ und X die in Anspruch 1 angegebene Bedeutung haben, mit Enaminverbindungen der allgemeinen Formel III, in welcher $R^2$ und $R^3$ die in Anspruch 1 angegebene Bedeutung haben, in Gegenwart von niederen aliphatischen Alkoholen mit 1 bis 4 Kohlenstoffatomen bei Temperaturen zwischen 40 und 100°C umsetzt, und anschließend 1 Gewichtsteil der so erhaltenen Verbindungen der allgemeinen Formel I mit 1 bis 20 Gewichtsteilen eines gut wasserlöslichen Hilfsstoffes und gegebenenfalls unter Verwendung weiterer üblicher Hilfs- und Trägerstoffe in ein Präparat mit verbesserter Bioverfügbarkeit überführt.